(19) **Europäisches Patentamt European Patent Office Office européen des brevets**

(11) **EP 2 059 814 B1**

(12) **EUROPEAN PATENT SPECIFICATION**

(45) Date of publication and mention
of the grant of the patent:
**18.08.2010  Bulletin 2010/33**

(51) Int Cl.:
*G01N 33/58* [(2006.01)]     *G01N 33/68* [(2006.01)]

(21) Application number: **07802859.4**

(22) Date of filing: **24.08.2007**

(86) International application number:
**PCT/EP2007/058810**

(87) International publication number:
**WO 2008/023055 (28.02.2008 Gazette 2008/09)**

(54) **METHOD FOR DETERMINING ANTIGEN CONTENT IN A SAMPLE USING DOUBLE IMMUNOHISTOCHEMICAL DETECTION**

VERFAHREN ZUR BESTIMMUNG DES ANTIGENGEHALTS IN EINER PROBE UNTER VERWENDUNG EINES IMMUNHISTOCHEMISCHEN DOPPELNACHWEISES

PROCÉDÉ PERMETTANT DE DÉTERMINER LE CONTENU ANTIGÈNE D'UN ÉCHANTILLON UTILISANT UNE DÉTECTION IMMUNOHISTOCHIMIQUE DOUBLE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **25.08.2006  PCT/EP2006/008376**

(43) Date of publication of application:
**20.05.2009  Bulletin 2009/21**

(73) Proprietors:
• **UNIVERSITE CATHOLIQUE DE LOUVAIN**
**1348 Louvain la Neuve (BE)**
• **Cliniques Universitaires Saint-Luc**
**1200 Bruxelles (BE)**

(72) Inventors:
• **MOULIN, Pierre**
**B-1050 Bruxelles (BE)**
• **GUIOT, Yves**
**B-1342 Limelette (BE)**
• **RAHIER, Jacques**
**B-5330 Assesse (BE)**

(74) Representative: **Brants, Johan P.E. et al**
**De Clercq Brants & Partners**
**Edgard Gevaertdreef 10a**
**9830 Sint-Martens-Latem (BE)**

(56) References cited:
**WO-A-00/31534**

• XUE YONG ET AL: "A novel immunoenzymatic technique to demonstrate multiple antigens in cells based on selective destaining of substrate deposits and its application in characterizing the immunophenotype of neuroendocrine cells in the human prostate epithelium" APPLIED IMMUNOHISTOCHEMISTRY, vol. 6, no. 2, June 1998 (1998-06), pages 69-76, XP008085355 ISSN: 1062-3345
• VALNES K ET AL: "PAIRED INDIRECT IMMUNOENZYME STAINING WITH PRIMARY ANTIBODIES FROM THE SAME SPECIES. APPLICATION OF HORSERADISH PEROXIDASE AND ALKALINE PHOSPHATASE AS SEQUENTIAL LABELS" HISTOCHEMICAL JOURNAL, SPRINGER-VERLAG, DORDRECHT, NL, vol. 16, no. 5, May 1984 (1984-05), pages 477-487, XP002977215 ISSN: 1567-2379
• GOWN A M ET AL: "AVIDIN-BIOTIN-IMMUNOGLUCOSE OXIDASE USE IN SINGLE AND DOUBLE LABELING PROCEDURES" JOURNAL OF HISTOCHEMISTRY AND CYTOCHEMISTRY, vol. 34, no. 3, 1986, pages 403-410, XP002405483 ISSN: 0022-1554
• TAYLOR C R ET AL: "Quantification of immunohistochemistry--issues concerning methods, utility and semiquantitative assessment II." HISTOPATHOLOGY. OCT 2006, vol. 49, no. 4, October 2006 (2006-10), pages 411-424, XP002405484 ISSN: 0309-0167

- ANONYMOUS: "Immunohistochemistry/Tissue Section Staining" INTERNET ARTICLE, [Online] 13 June 2004 (2004-06-13), XP002405485 Retrieved from the Internet: URL:http://web.archive.org/web/20040613113 333/http://www.bdbiosciences.com/pharminge n/protocols/Tissue_Section_Staining.shtml> [retrieved on 2006-11-02]
- YOSHIKO NAKAE ET AL: "A new technique for the quantitative assessment of 8-oxoguanine in nuclear DNA as a marker of oxidative stress. Application to dystrophin-deficient DMD skeletal muscles" HISTOCHEMISTRY AND CELL BIOLOGY ;, SPRINGER-VERLAG, BE, vol. 124, no. 3-4, 1 September 2005 (2005-09-01), pages 335-345, XP019343075 ISSN: 1432-119X
- WAHLBY CAROLINA ET AL: "Sequential immunofluorescence staining and image analysis for detection of large numbers of antigens in individual cell nuclei" CYTOMETRY, vol. 47, no. 1, 1 January 2002 (2002-01-01), pages 32-41, XP002456985 ISSN: 0196-4763

## Description

### FIELD OF THE INVENTION

The present invention relates to the field of immunohistochemistry and immunohistodensitometry. The present invention provides a method for determining antigen content in a region of interest of a sample. The method comprises a double immunohistochemical detection of the antigen to be quantified and the region of interest and image-based computerized analysis thereof by the application of a specific algorithm. The present invention further relates to a kit for determining antigen content in a region of interest of a sample.

### BACKGROUND OF THE INVENTION

Immunohistochemistry ("IHC") is the localization of antigens in tissue sections by the use of labeled antibodies as specific reagents through antigen-antibody interactions that are visualized by a marker such as fluorescent dye, enzyme, radioactive element or colloidal gold. Since immunohistochemistry involves specific antigen-antibody reaction, it has apparent advantage over traditionally used special and enzyme staining techniques that identify only a limited number of proteins, enzymes and tissue structures. Therefore, immunohistochemistry has become a crucial technique which is widely used in many medical research laboratories as well as clinical diagnostics.

Immunohistochemistry includes a family of techniques based on the use of a specific antibody, wherein antibodies are used to specifically target antigens inside or on the surface of cells. The antibody typically contains a marker that will undergo a biochemical reaction, and thereby experience a change of color, upon encountering the antigens. In some instances, signal amplification may be integrated into the particular protocol, wherein a secondary antibody, that includes the marker stain, follows the application of a primary specific antibody. In both hybridization and immunolabeling studies, chromogens of different colors are used to distinguish among the different markers.

Visualisation and evaluation of the coloring reactions may be done using a microscope. Tissue samples and cell preparations are visually inspected by pathologists under several different conditions and test procedures with use of microscopes. Based on these visual inspections, determinations concerning the tissue or cellular material can be deduced. Most morphologists, including pathologists, perform semi-quantitative evaluation of immunohistochemical markers depending on their training and level of expertise, which, therefore, remains subjective. In clinical pathology, confirmation of a diagnosis can only be obtained by the advice of another pathologist. Similarly, it's by comparing the evaluation of several observers that the consistency of scoring of immunohistochemical staining intensities is established in research studies.

In the past years, there have been improvements in the production of images themselves such as confocal, two-photon or virtual microscopy and many developments have been dedicated to digital image treatment through computer processing.

Images need to be acquired and stored in an appropriate way to obtain quality digital material. Furthermore, before measuring any feature the image must be segmented. Segmentation is the process of dividing an image into parts that distinguish structures of interest from non relevant background. These parts are called objects. They can be referred to as parts of the image bearing a sufficient labeling or to reference areas. The result of segmentation is a *binary* image where pixels corresponding to objects are set to 1 whereas others are set to 0. Automatic segmentation is one of the most difficult tasks in image analysis and implies the use of very sophisticated mathematical methods. The counter part of automatic segmentation is interactive segmentation, whereby an observer is asked to define objects or areas. Although easy to set up, the latter practice falls down on its poor inter-observer reproducibility, especially when comparing naïve users with others who are familiar with the experimental material.

Optical density or absorbance measurement on digital images allows quantifying antigen concentration on slides. However, any quantification requires a reference (segmentation) system, and this entrains a problem in immunohistodensitometry since the reference is an area which is not easy to determine. Results of immunohistochemical analyses are strongly dependent on the accuracy of the choice of reference area, and mistakes on the reference area calculation may yield incorrect quantification results.

Prior art methods have been disclosed wherein use is made of an immunomarker which is selective for cell components of interest, and a histochemical marker which counterstains a specific area of the cell. However, using such techniques, the reference area is defined according to the subcellular distribution of the marker, such as the nucleus, cytoplasm or even the whole cell. Segmentation of an immunohistochemical signal upon the corresponding grey-level in an image leads to serious problems for example when the cell components of interest are heterogeneously distributed in the reference area or in the cell. In such methods, the area of the segmented signal does not fit the actual reference area which is expectedly larger. This results in inappropriate optical density values and inappropriate quantification when comparing analysis results of two or more cells, having a slightly different volume of reference area.

Another drawback of prior art methods is that interference may occur between the counterstain obtained with the histochemical marker and the immunomarker signal. Both markers are generally chosen such that their detection wavelengths are sufficiently distant from each other to avoid substantial interference when taking the images with the aid of two filters, one specific for the wavelengths of the immunomarker, the other specific for the wavelengths of the histochemical marker. However, up to now, no markers have been found which completely exclude interference.

Xue et al. (Applied Immunohistochemistry 6(2), 69-76, 1998) describes an immunoenzymatic technique to demonstrate multiple antigens in cells based on sequential double staining and selective destaining of substrate deposits.

In view of the above, it is clear that there remains a need in the art for an improved method for performing immunohistochemical analysis. More in particular, there remains a need in the art for an improved method for determining the optical density or absorbance measurement on digital images of cell or tissue samples, and thus for determining the content of cell components of interest, in particular antigen(s), in a sample region.

It is therefore an object of the present invention to provide an improved method and kit for determining antigen content in a cell or tissue sample. In particular, the present invention aims to provide an improved method and kit for making multi-modal (digital) images of cell or tissue samples on which morphometric and densitometric analyses may be performed. The present invention also aims to provide a method wherein determination of a reference (segmentation) area in the acquisition of digital images is improved.

## SUMMARY OF THE INVENTION

In a first aspect, the present invention relates to an improved method as claimed in present claim 1, for determining antigen content in a specific cell or tissue sample or compartment which overcomes at least some of the previously mentioned problems of the prior art. The present method is at least in part based on an improved determination of a correct and suitable reference area in digital images taken from immunohistochemical samples.

In particular the present invention provides a method for determining antigen content in a region of interest of a sample comprising the steps of:

a) providing a slide containing said sample,
b) submitting said sample to immunomarking comprising the steps of:
b1) optionally submitting said sample to a stabile immunomarking by incubating said sample with an antibody-chromogen system I thereby forming a stabile chromogenic product not soluble in buffering solutions and mounting media, and
b2) submitting said sample to a labile immunomarking by incubating said sample with an antibody-chromogen system II thereby forming a labile chromogenic product which precipitates under aqueous conditions and which can be solubilized in alcohols,
c) acquiring first image(s) of said sample containing said labile chromogenic product optionally in combination with said stabile chromogenic product, thereby defining a reference area,
d) eluting said labile chromogenic product using an elution product selected from the group consisting of alcohols or hydrophobic solvents such as toluol,
e) provided that step b1) was not carried out, submitting the sample of step d) to a stabile immunomarking by incubating said sample with an antibody-chromogen system I thereby forming a stabile chromogenic product;
f) acquiring second image(s) of said sample containing said stabile chromogenic product,
g) processing said first and second image(s) by superimposing the first image(s) on the second image(s) or by superimposing a mask extracted from said first image(s) on the second image(s), and
h) determining antigen content of said sample within the defined reference area.

In an embodiment, incubation of said sample with an antibody-chromogen system I, said antibody-chromogen system I comprising a primary antibody AbI directed against said antigen, a detection system I able to detect said primary antibody AbI, and a chromogenic reagent I suitable for being converted by said detection system I into a stabile chromogenic product,

said incubation comprising the subsequent steps of:

- incubating the sample with the primary antibody AbI of the antibody-chromogen system I
- adding said detection system I and said chromogenic reagent I to said incubated sample thereby forming a stabile chromogenic product, and
- optionally removing bound primary antibody AbI by acidic elution.

In an embodiment, incubation of said sample with an antibody-chromogen system II, 1,
said antibody-chromogen system II comprising a primary antibody AbII directed against said antigen, a detection system II able to detect said primary antibody AbII, and a chromogenic reagent II suitable for being converted by said detection system II into a labile chromogenic product,
said incubation comprising the subsequent steps of:

- incubating said sample with the primary antibody AbII of the antibody-chromogen system II, and
- adding said detection system II and said chromogenic reagent II to said incubated sample thereby forming a labile chromogenic product.

In an embodiment, said images are acquired by:

- mounting the slide containing the sample on a computer-controlled optical microscope equipped with a digital camera and a motorized stage,
- acquiring digital images of different areas of said sample, and
- storing said images on a suitable storage means.

In an embodiment, said antigen content is determined by calculating optical density (SOD) of the superimposed image using an algorithm,
wherein
Im corresponds to the compound image of $Im_1$ and $Im_2$
$Im_1$ corresponds to an image with a labile and optionally also a stabile chromogenic product,
$Im_2$ corresponds to an image with a labile chromogenic product,
A corresponds to a region covered by the labile immunomarking,
$O_1$ corresponds to a region covered by the stabile immunomarking, and
let $a[m,n] \in A$, $o[m,n] \in O_1$ be pixels of image Im,
and $d[m,n]$, be the grey level of pixel $a[m,n]$ in image $Im_2$

then surface density of the labile immunomarking in the stabile immunomarking is given by: $\dfrac{|A|}{\sum |O_i|}$

the mean grey level of a subset of pixels defined in image $Im_2$ is defined by :

$$m_o = \frac{\sum\limits_{i} \sum\limits_{[m,n] \in O_i} d[m,n]}{\sum\limits_{i} |O_i|}$$

the mean grey level of pixels in image $Im_2$ is computed from said subset of pixels defined in image $Im_2$ and is referred to pixels defined in $Im_1$:

$$m_d = \frac{\sum\limits_{[m,n] \in A} d[m,n]}{|A|} \ .$$

The present method involves a double immunohistochemical detection, whereby two distinct immunohistochemical detection procedures of proteins (antigens) and/or nucleic acid sequences, and/or sugars are applied. A stabile immunostaining procedure is carried out for performing density measurements. Density detection is performed in a single immunostaining step. The introduction of an additional, in a particular a labile immunostaining step in the present method allows a more precise determination of the reference area, which is very advantageous in cases where the variation of antigen content is accompanied by modification of its distribution in the cell, or when variations of cell volume are susceptible of modifying the evaluation of a membrane-bound signal. With the insight to better show the characteristics of the invention, some preferred embodiments and examples are described hereafter referring to the enclosed figures.

## BRIEF DESCRIPTION OF THE FIGURES

**FIG. 1-3** represent flow charts illustrating methods according to the present invention.

**FIG. 4** represents the relationship between concentration of antibodies and image signal intensity.

**FIG. 5** is a schematic representation of the processing of images.

**FIG. 6A, B** and **C** schematically represent an image $Im_1$, image $Im_2$ and a composed image Im, respectively.

**FIG. 7A, B,** and **C** illustrate histogram of the probability functions p[i], p[o] and p[a] of pixels from $Im_2$, $O_i$ and A respectively.

**FIG. 8** demonstrates the density measurements of insulin immunostaining in islets from control and glibenclamide-treated rats with different reference areas. A) Specific absorbance of labeling using primary antibody diluted at 1/1000 and 1/2000; B) Specific absorbance of islets from treated rats, relative to controls in identical conditions (set to 100%), using primary antibody at 1/1000 and 1/2000 ($p < 0.05$).

**FIG. 9** shows GLUT2 expression in islet β cells during life, measured by the direct and referred methods. Differences are statistically significant between each group ($p<0.05$).

**FIG. 10** illustrates the determination of insulin (antigen) specific optical density by three different methods as well as the impact of an inexperienced user.

## DETAILED DESCRIPTION OF THE INVENTION

Many proteins are unequally distributed in a cell. Some of them are only found in the plasma membrane whereas other are bound to specific organelles or contained in specific subcellular cisterns, or even in the nucleus. Evaluation of the abundance of these proteins can be carried out by immuohistodensitometry, a method by which a protein is detected by an immunohistochemical procedure leading to chromogen deposit (further referred as to chromogenic product), the optical density of which is then measured. Immunohistodensitometry gives the optical density of the chromogenic product as it is measured in a particular area of the tissue sample.

**Method**

The present invention is directed to a method for determining antigen content in a region of interest of a sample.

The term *"antigen"* as used herein refers to a substance that stimulates an immune response, especially the production of antibodies. Antigens usually are proteins or polysaccharides, but can be any type of molecule, including nucleic acid probes used to detect a specific DNA or RNA sequence.

The sample according to the invention may include a tissue or a cell, for instance any sample suitable for pathological examination, including frozen or fixed and embedded tissue, whatever the fixative such as formalin or Bouin's solution, and/or whatever the embedding media such as paraffin or resin. The sample according to the present invention may be cut into sections and mounted on slides which are suitable for microscopic examination, e.g. glass or plastic slides.

The term "immunohistochemical system" as used herein refers to a system, which is able to detect the presence of a certain antigen in a sample. In a preferred embodiment, the present revelation system is further able to amplify the signal detecting the antigen. In certain embodiments, the term "immunohistochemical system" is used herein as synonym for antibody-chromogen system, or antibody-chromogen composition. In a preferred embodiment, the immunohistochemical system comprises at least three components, which are able to interact with one another. Said components comprise at least one antibody directed against an antigen of interest, a detection system provided with an enzyme and a chromogenic reagent, the latter being the substrate for the enzyme. It shall be clear that said at least three components of the system can be added separately, simultaneously or sequentially in the methods of the present invention.

The invention comprises a method for determining antigen content in a region of interest of a sample comprising the steps of:

a) providing a slide containing said sample,

b) submitting said sample to immunomarking comprising the steps of:

b1) optionally submitting said sample to a stabile immunomarking by incubating said sample with an antibody-chromogen system I thereby forming a stabile chromogenic product not soluble in buffering solutions and mounting media, and

b2) submitting said sample to a labile immunomarking by incubating said sample with an antibody-chromogen system II thereby forming a labile chromogenic product which precipitates under aqueous conditions and which can be solubilized in alcohols,

c) acquiring first image(s) of said sample containing said labile chromogenic product optionally in combination with said stabile chromogenic product, thereby defining a reference area,

d) eluting said labile chromogenic product using an elution product selected from alcohols or hydrophobic solvents such as toluol,

e) provided that step b1) was not carried out, submitting the sample of step d) to a stabile immunomarking by incubating said sample with an antibody-chromogen system I thereby forming a stabile chromogenic product;

f) acquiring second image(s) of said sample containing said stabile chromogenic product,

g) processing said first and second image(s) by superimposing the first image(s) on the second image(s) or by superimposing a mask extracted from said first image(s) on the second image(s), and

h) determining antigen content of said sample within the defined reference area.

According to the present invention, a sample is submitted to a stabile and a labile immunomarking. The term *"stabile"* immunomarking, according to the invention relates to an immunomarking procedure using an antibody-chromogen system which is capable of forming a stabile chromogenic product. The term *"labile"* immunomarking according to the invention relates to an immunomarking procedure using an antibody-chromogen system which is capable of forming a labile chromogenic product. The term *"first images"* according to the invention relates to images of said sample containing at least the labile chromogenic product. Such sample may thus comprise either only a labile chromogenic product or a labile and a stabile chromogenic product. The term *"second images"* according to the invention relates to images of said sample containing only a stabile chromogenic product. The term *"composed image"* as used herein refers to an image that has been obtained by processing the first and second image(s). Processing may be performed in different ways as further explained below.

The term *"stabile chromogenic product"* refers to a chromogenic product or deposit which is not soluble in buffering solutions and mounting media. The term *"labile chromogenic product"* refers to a chromogenic product which precipitates under aqueous conditions, e.g. in water-based buffers, but which can be solubilized in alcohols.

Hereafter, *"labile"* with reference to a chromogen is intended to mean that the said chromogen is chosen such that it can be eluted by a specific solvent such as ethanol, methanol or toluol. Labile may be considered as a synonym for "capable of being eluted or being elutable".

Hereafter, "stabile" with reference to a chromogen is intended to mean that the said chromogen is chosen such that it is not affected by the subsequent steps of the immunohistochemical procedure including those that aim to the solubilization of the labile chromogene. Stabile may be considered as a synonym for "not capable of being eluted or being non-elutable".

In one embodiment, the method involves the steps of:

a) providing a slide containing said sample,

b) submitting said sample to a stabile immunomarking by incubating said sample with an antibody-chromogen system I thereby forming a stabile chromogenic product not soluble in buffering solutions and mounting media,

c) submitting the sample of step b) to a labile immunomarking, by incubating said sample with an antibody-chromogen system II thereby forming a labile chromogenic product which precipitates under aqueous conditions and which can be solubilized in alcohols;

d) acquiring one or more first image(s) of the sample obtained in step c) thereby defining a reference area,

e) eluting said labile chromogenic product using an elution product selected from alcohols or hydrophobic solvents such as toluol,

f) acquiring one or more second image(s) of the sample obtained in step e),

g) processing said first and second image(s) by superimposing the first image(s) on the second image(s) or by superimposing a mask extracted from said first image(s) on the second image(s), and

h) determining antigen content of said sample within the defined reference area.

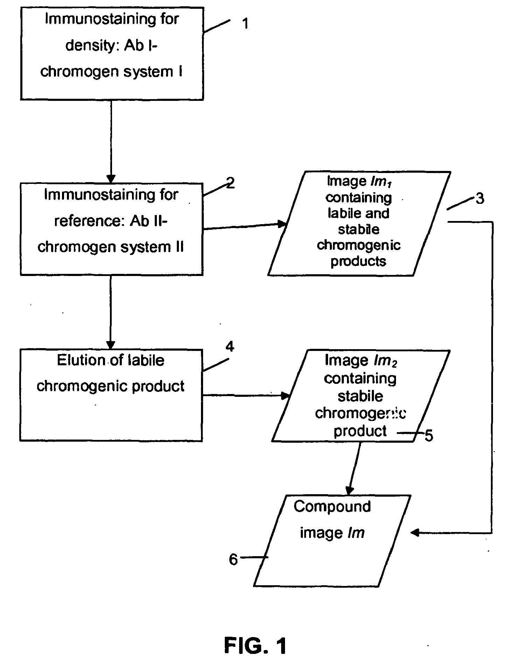

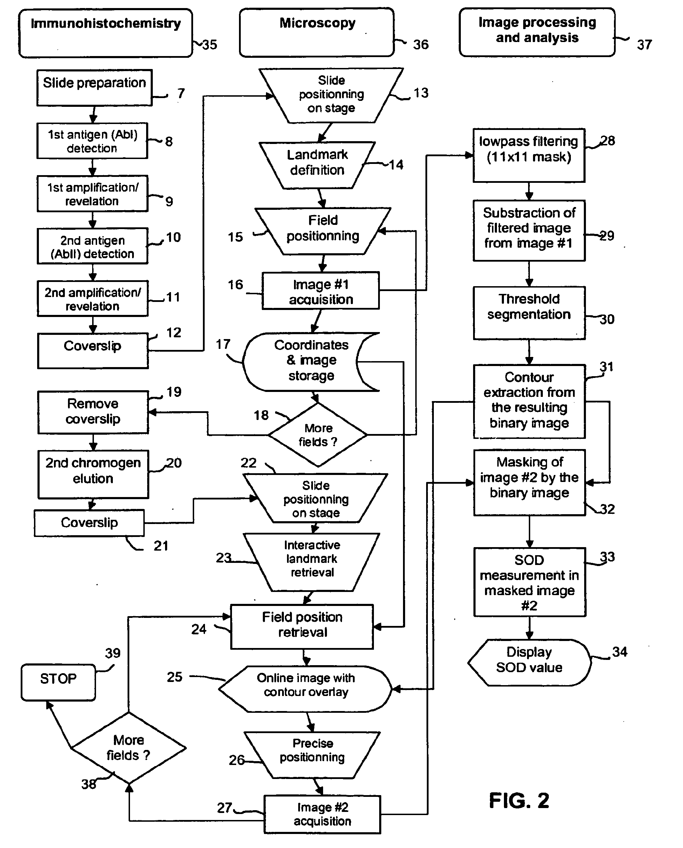

In such method, by eluting the labile chromogenic product resulting from the labile immunostaining step before acquiring the second image(s), the signal used for defining the reference area is no longer present when taking the second image, thereby preventing interference of the labile immunomarking signal with stabile immunomarking signal. Said method is schematically represented in **FIG. 1** (see below). A more detailed flow chart of this method is illustrated in **FIG. 2** (see below).

In an alternative embodiment, the method involves the steps of:

a) providing a slide containing said sample,

b) submitting said sample to a labile immunomarking by incubating said sample with an antibody-chromogen system II thereby forming an labile chromogenic product which precipitates under aqueous conditions and which can be solubilized in alcohols,

c) acquiring one or more image(s) of the sample obtained in step b) thereby defining a reference area,

d) eluting said labile chromogenic product using an elution product selected from alcohols or hydrophobic solvents such as toluol,

e) submitting the sample of step d) to a stabile immunomarking, by incubating said sample with an antibody-chromogen system I thereby forming a stabile chromogenic product;

f) acquiring one or more second image(s) of the sample obtained in step e),

g) processing said first and second image(s) thereby obtaining a compound image, and

h) determining antigen content of said sample within the defined reference area.

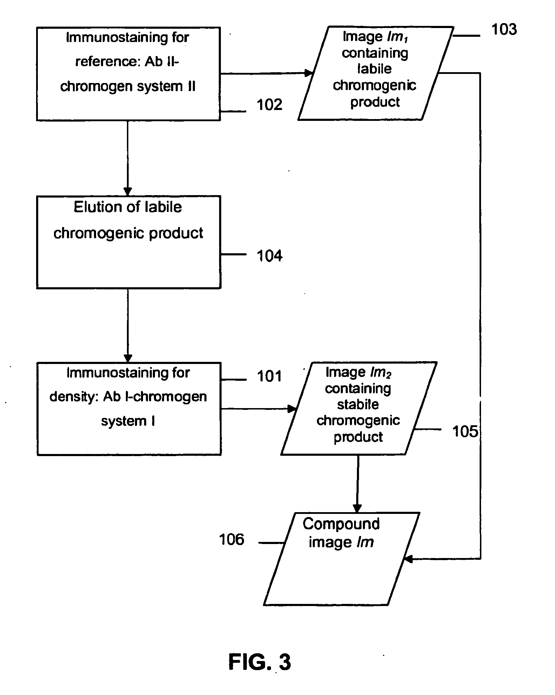

Said method is schematically represented in **FIG. 3** (see below).

The first and second image(s) are processed in order to obtain a composed image. The first and second images may be stored in suitable storage means (databases).

In one embodiment, processing of said first and second image(s) comprises superimposing the first image(s) on the second image(s). The method may thus involve actual superimposition of stored first and second images.

In another embodiment, processing of said first and second image(s) comprises extracting a mask from said first image(s) and superimposing said mask on the second image(s). In accordance with the present invention, the mask from said first image(s) is obtained by drawing a binary image which covers regions of interest in said first image and extracting the contours of said binary image. A binary image contains only black and white pixels. It is represented as a logical array of 0's and 1's, which is usually converted to a numerical array of 0's and 255's, respectively. The contours of the binary mask extracted from stored first images are calculated and visualized as an overlay in the online image so that the overlaid contours of the binary mask from first images can be aligned with the second image online when putting the sample under the microscope, after which the second image is acquired. The term "*contours*" as used herein refers to the outlined detected area in first image(s), i.e. the boundaries of the mask. Image masking is a versatile technique in which certain areas of interest are highlighted. Masking is useful for defining the region(s) of an image in which statistics are required. A mask image is an image which contains the value 255 in the area of interest and zero elsewhere.

The following steps are used to create a mask image. In the present approach, areas of interest are determined in the first image(s) and a mask is drawn from them (i.e. a binary image covering these regions of interest. Next, contours are extracted. This can be done either by erosion whereby the boundary is given by $\beta(A)= A - (A\Theta B)$ wherein A is the subset of identified areas in the image and B is a structuring element, usually a cross in a 3x3 matrix. Contours can also be extracted by edge detection, whatever the type of derivative operator (Gradient or Laplacian) which is used.

In accordance with the present processing step; multi-modal image(s) is (are) formed displaying the signal of a stabile immunomarking restricted to a reference area defined by the signal of the labile immunostaining. The immunomarking with the stabile chromogenic product allows density measurements while the immunomarking providing a labile chromogenic product, is used to define the reference area which improves the reliability of the quantitative analysis. Using such co-registration process the immunolabeling absorbance (optical density) can then be computed. Performing detection of two sequential immunomarkings in this way, the computed absorbance allows further determination of the content of the antigen of interest more precisely in the tissue labled by the labile immunomarking.

Antibody-chromogen systems used in the immunomarking steps are made out of several components which can be added separately, sequentially or simultaneously during the marking process.

An antibody-chromogen system I which is capable of forming a stabile chromogenic product preferably comprises: a primary antibody AbI directed against the antigen, a detection system I for detecting said primary antibody AbI, and a chromogenic reagent I suitable for being converted by said detection system I into a stabile chromogenic product.

An antibody-chromogen system II which is capable of forming a labile chromogenic product preferably comprises: a primary antibody AbII directed against a reference antigen, a detection system II for detecting said primary antibody AbII, and a chromogenic reagent II suitable for being converted by said detection system II into a labile chromogenic product.

The stabile immunomarking step preferably comprises the subsequent steps of:

- incubating the sample with the primary antibody AbI of the antibody-chromogen system I
- adding said detection system I and said chromogenic reagent I to said incubated sample thereby forming a stabile chromogenic product, and

- optionally removing bound primary antibody AbI by acidic elution.

The labile immunomarking step preferably comprises the subsequent steps of:

- incubating said sample with the primary antibody AbII of the antibody-chromogen system II, and
- adding said detection system II and said chromogenic reagent II to said incubated sample thereby forming a labile chromogenic product.

Images are acquired according to the present invention by mounting the slide containing the sample on a computer-controlled optical microscope equipped with a digital camera and a motorized stage. Digital images of different areas of said sample are acquired and the acquired images are preferably stored on a suitable storage means.

According to the invention, digital images can be acquired by monochrome or color CCD cameras. Multi-spectral imaging represents a possible refinement of acquisition. Therefore in one embodiment, the present method is characterized in that images are acquired by monochrome or color CCD cameras. In another embodiment, the present method is characterized in that images are acquired by means of a multi-spectral imaging technique.

Multi-spectral imaging is a method to acquire microscopic images. It can improve the specificity of detecting a given chromogen but does not preclude the use of sequential elution. Therefore, the present invention further relates to the use of multi-spectral imaging for acquiring digital images in a method of the present invention. More in particular, first and/or second images representing one or both chromogenic products can according to the present invention by acquired by multi-spectral imaging to improve the specificity of signal detection.

Referring now to **FIG. 1**, some steps of a method according to the invention are schematically illustrated. In a first immunomarking, step in box 1, a sample provided on a microscopic slide is submitted to an immunomarking for determining density. Thereby the sample is brought into contact with an antibody (AbI)-chromogen system I capable of forming a stabile chromogenic product and a stabile chromogenic product, preferably a deposit, is formed. Preferably, the antibody-chromogen system I comprises a primary antibody (AbI) which is directed against the antigen, a detection system I for detecting said primary antibody AbI and a chromogenic reagent I suitable for being converted by said detection system I into a stabile chromogenic product. According to the specifications of the primary antibody, two procedures can be used: for instance overnight incubation at 4°C or 2h-incubation at 37°C. The former is suitable for manual processing whereas the latter matches the optimal condition in automated immunostainers. Then, the detection system I and the chromogenic reagent I are added to the incubated sample, either simultaneously or separately.

In selected conditions, antibody AbI and the associated detection system I can be removed after precipitation of chromogenic products without affecting the chromogenic deposit itself. Removal of unbound antibody AbI is done by rinsing. Removal of bound primary antibody AbI can be done using appropriate eluting solutions, such as but not limited to acidic solutions. Elution can for instance be done with a 0.1N HCl aqueous solution during 1h at room temperature. This elution step has to be done carefully to prevent partial or complete removal of the stabile chromogenic product. Removal of bound primary antibody AbI is preferably done prior to primary antibody AbII incubation, and preferably in case the primary antibody AbI occupies the same epitope and therefore prevents the primary antibody AbII of accessing its binding site. However, removal of bound primary antibody AbI can also be done prior to primary antibody AbII incubation, in cases wherein the primary antibody AbI and the primary antibody AbII are different.

Then a labile immunomarking step is performed in box 2 using an antibody-chromogen system II yielding a labile chromogenic product. This marking is applied to detect the antigen of interest or another antigen present in the cell or tissue. This antibody-chromogen system II preferably comprises a primary antibody (AbII) directed against a reference antigen, a detection system II for detecting said primary antibody AbII and a chromogenic reagent II suitable for being converted by said detection system II into a labile chromogenic product.

In a next step the slides containing the stained sample are mounted in medium such that the labile chromogen deposit is preserved. Examples of suitable media include but are not limited to water-based mounting media for Fast-red, a glycerin solution, etc... Glycerin is used in water-based mounting medium mainly to increase viscosity and adherence.

Subsequently, the slides are placed on a computer-controlled optical microscope equipped with a digital camera and a motorized stage. Digital images of different areas of said sample are acquired (images $Im_1$) as shown in box 3, and acquired images are stored on a suitable storage means (not shown).

In a next step, the method involves elution in box 4 of the labile chromogenic product. The elution product (elution product X) must be efficient in removing the labile chromogenic product without altering the signal of the stabile chromogenic product of the first immunomarking. Preferably the method involves elution of the labile chromogenic product by immersing said sample in absolute ethanol.

Then, the eluted slides are mounted on the above-indicated optical microscope, digital images of different areas of the sample are acquired (images $Im_2$) as shown in box 5, the images are matched with the images of sample containing the both chromogens (box 3).

First and second images are then further processed, as explained above, in order to obtain a composed image as indicated in box 6.

FIG. 2 illustrates an example of a method according to the invention, wherein a slide containing a tissue sample is prepared in box 7 and submitted to sequential incubations of a primary antibody AbI in box 8, corresponding detection system yielding the stabile chromogenic product in box 9, optional elution of the antibody AbI (not shown) before incubation with the primary antibody AbII in box 10 and detection yielding deposit of labile chromogenic product in box 11. The slide is mounted in box 12 and placed onto an optical microscope equipped with a digital camera and a motorized stage, both controlled by a computer which preferably implements as illustrated by the microscopy procedure under box 36 the following protocol:

1. warn the operator to be sure that the slide is correctly installed and locked onto the motorized stage in a given orientation (e.g. label on the right) as illustrated in box 13,

2. locate one particular point of the slide that produces an image independently from the labeling (e.g. label border) as illustrated in box 14,

3. field positioning as illustrated in box 15 or (optionally) run a meander scanning of the slide in order to provide a low-magnification overview of the slide where areas of interest can be located more easily,

4. sequentially acquire and store images from working resolution fields of interest, either retrieved from overview image or selected by the operator (as illustrated in box 16), and storing the spatial coordinates thereof into a data file containing a reference to the slide (as illustrated in box 17),

5. When all fields of interest have been processed (Operator warning: as illustrated in box 18), remove the slide from the microscope and go back to immunohistochemical procedure as illustrated under box 35,

6. Remove coverslip (as illustrated in box 19) and elute labile chromogenic product (as illustrated in box 20), then remount (as illustrated in box 21),

7. End of the immunohistochemical protocol (as illustrated in box 35) and continuation of the microscopy protocol (as illustrated under box 36): place the slide onto the optical microscope described earlier,

8. Warn the operator to be sure that the slide is correctly installed and locked onto the motorized stage in a given orientation (e.g. label on the right)( as illustrated in box 22) and locate the particular point of the slide which was used as reference in step 2 (e.g. label border), as illustrated in box 23,

9. Recover previously stored spatial positions for the given field of the given slide (as illustrated in box 24) and move the motorized stage to the said coordinates, where auto focus is ran or the operator asked to do so,

10. Image processing and analysis is performed as indicated under box 37. Display the online image produced by the camera with the superimposition of the contours (as illustrated in box 31) of a binary mask extracted by thresholding (as illustrated in box 30) from filtered image (as illustrated in boxes 28 and 29) of the same field, as taken during step 4. The image is filtered in box 28, possibly processed in box 29 and segmented in box 30 so that the result is a binary image representing for instance, the reference area from which the borders of objects are extracted (e.g. Sobel gradient filtering) (as illustrated in box 31).

11. The contours are displayed as an overlay on the online image in box 25, which allows the operator to finely match the online image with the filtered stored image by moving the stage interactively (as illustrated in box 26) prior to image grabbing and storing in box 27.

12. Finally, image #2 as acquired in step 11 is masked by binary image resulting from step 10 in box 32 in order to perform measurements in box 33 and display or store the results in box 34,

13. Go to step 9 until no more fields are available in box 38 and then stop in box 39.

Referring now to FIG. 3, another method according to the invention is illustrated. During a labile immunomarking step in box 102, a sample provided on a microscopic slide is brought into contact with an antibody (AbII)-chromogen system II whereby a labile chromogenic product, preferably a deposit, is formed. Immunomarking can be performed as explained in the method given above. Then, digital images of different areas of said sample are acquired (images $Im_1$) as shown in box 103, and acquired images are stored on a suitable storage means (not shown). In a next step, the method involves elution in box 104 of the labile chromogenic product. Elution can be performed as explained in the method given above. In a next step, a stabile immunomarking is performed in box 101 using an antibody (AbI)- chromogen system I. Digital images of different areas of the sample are acquired (images $Im_2$) as shown in box 105. The images are matched with the images of sample containing the chromogen of box 103. First and second images are then further processed in order to obtain a composed image as indicated in box 106.

**Algorithm**

Optical density or absorbance measurement on digital images allows to quantify antigen concentration on slides. The principle is derived from Beer-Lambert formula (equation 1):

$$A = \log \frac{I_0}{I} = \varepsilon c l \quad \textbf{(equation 1)}$$

The extension of this principle to immunohistochemistry assumes that each pixel of a digital image can be considered as similar to a spectrometry tube. Unlike spectrometry tubes, which are simple containers with known optical characteristics, histological slides are an arrangement of elements with different and somehow unpredictable optical characteristics. To prevent these elements from altering the measurement, their own absorbance should be subtracted from the final value, namely, the specific optical density (equation 2)

$$SOD = \log \frac{I_0}{I_{T+L}} - \log \frac{I_0}{I_T} = \log \frac{I_T}{I_{T+L}} \quad \textbf{(equation 2)}$$

wherein $I_0$, $I_T$, and $I_{T+L}$ are the intensity of the incoming light, transmittance of unstained tissue and glass, and transmittance of labeling, tissue and glass, respectively.

Incoming light ($I_0$) is partially absorbed by the glass slide and coverslip but also by the unstained tissue, independently from the chromogen deposit. The light intensity at a given point of the slide can be read as a function of the grey value of the corresponding pixel (equitation 3).

$$I = \alpha . GV \quad \textbf{(equation 3)}$$

representing the relationship between light intensity (or transmittance) at a given spot and the grey level value of the corresponding pixel. I is the intensity of the incident light, GV is the grey level of the pixel and $\alpha$ is constant which is independent from GV.

Therefore, SOD can be computed using the grey value of pixels corresponding to stained objects, and corrected by the background grey value, which is measured in the unstained tissue. In appropriate conditions of immunolabeling, the specific optical density reflects the antigen content in the corresponding unitary area on the histological slide.

In according with the present invention, antigen content is determined by calculating optical density (SOD) of the composed image using a specific algorithm. Below the algorithm is further explained.

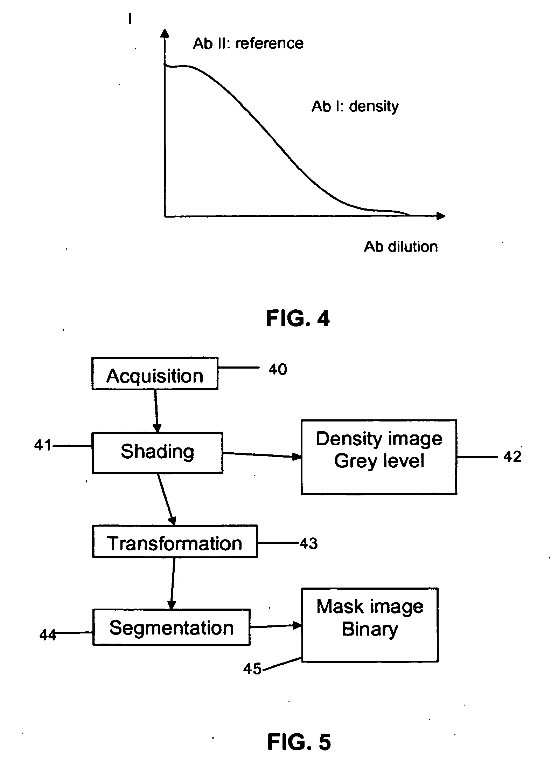

Referring to **FIG. 4,** for density measurements with antibody AbI, the concentration of the primary antibody is chosen such that stall variation in antigen content will result in linearly related variation in signal intensity. The consequence is that zones that are poor in antigen are weakly labeled and possibly missed by the segmentation process. Conversely, for reference area determination with antibody AbII, the concentration of the primary antibody is chosen high enough to even label zones of poor antigen content with a sufficient intensity so that they are included in the segmentation process even though saturation of signal intensity prevents reliable measurements thereof.

Segmentation refers to assigning image pixels to regions or objects, in accordance with predetermined criteria (homogeneity predicate). As indicated in **FIG. 5,** images are acquired in box 40 and segmentation processes as shown in box 44 can occur on any transformation of images, that is transformation as indicated in box 43 of the original image that increases the accuracy of segmentation. Grey level measurements as indicated in box 42 must be performed on original images or images where shading correction was applied as shown in box 41. The method by which images are produced - the interaction between objects in real space, the illumination, and the camera - frequently leads to situations where the image exhibits significant shading across the field-of-view. In typical micrographs the image might be bright in the centre and decrease in brightness as one goes to the edge of the field-of-view. The shading might be caused by non-uniform illumination, or even dirt and dust on lens surfaces. In general this shading effect is to be eliminated for subsequent processing and especially for image analysis. The binary mask image obtained after segmentation, as shown in box 45, defines which pixels of the grey level density image of box 42 must be evaluated.

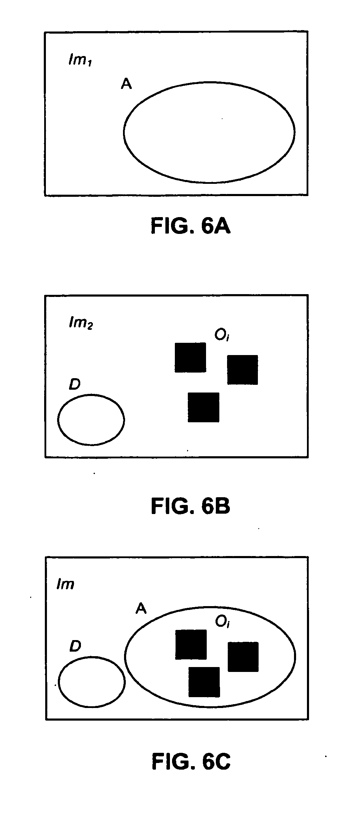

From the immunostaining designed to evaluate the maximum area which contains antigen II, the segmentation as represented in **FIG. 6A,** of image $Im_1$, is done: where: $Im_1$ is a greylevel image, of the staining with AbII, and A is the region covered by the labile chromogen. A stabile immunostaining might still be visible «under» the labile immunohistochemical signal, or not, depending on the applied method.

From the immunostaining designed to evaluate antigen density and distribution, the segmentation as represented in **FIG. 6B** of image $Im_2$ is done: where

$Im_2$ is a greylevel image adapted to density measurement, superimposabe on $Im_1$ $O_i$ are the regions covered by the stabile chromogenic product,

D is a region where a densitometric reference is measured.

D is usually defined interactively by the operator; $O_i$ are defined as subset of pixels of image Im which are selected upon their grey level by reference to a threshold value either on the original image or any transformation thereof.

It is noted that image $Im_2$ only contains one chromogenic product (i.e. that designed for densitometry), but $Im_1$ can either contain both labile and stabile chromogenic products, or only a single labile chromogenic product.

From the surperimposition of both first and second images the system as illustrated in **FIG. 6C** is build; where Imis a compound image with two modalities corresponding to $Im_1$ and $Im_2$,

A is the region covered by the labile immunostaining,

$O_i$ are the regions convered by the stabile immunostaining,

D is a region where densitometric reference is measured.

A and $O_i$ are partitions of the entire image $Im$,

That is A and $O_i$ are subsets of connected pixels of $Im$,

$O_i$ are subsets of A: $O_i \subseteq A$,

$O_i$ are not connected: $O_i \cap O_j = \phi$ for *all* $i \neq j$,

A homogeneity predicate H(R) is a function that takes a region R and returns true or false according to the pixel properties,

A is defined by a homogeneity predicate in $Im_1$: $H_1(A) = TRUE$,

$O_i$ are defied by a homogeneity predicate in $Im_2$: $H_2(O_i) = TRUE$ for $i = 1,2,...,n$

$H_2$ might differ for each subset $O_i$.

Therefore, the subset of interest is define on by A on $Im_1$ and measured on $Im_2$, where otherwise, the subset of pixels of interest would be reduced to $\{O_i\}$,

Let $a[m,n] \in A$, $o[m,n] \in O_i$ be pixels of image $Im$,

and $d[m,n]$, be the grey level of pixel $a[m,n] \in A$, in image $Im_2$

then the surface density, i.e. the ratio between the area of objects $O_i$ and that of the reference area A, of the labile immunomarking in the stabile immunomarking is given by:

$$\frac{|A|}{\sum |O_i|}$$

the mean grey level of a subset of pixels defined in image $Im_2$ is defined by :

$$m_o = \frac{\sum_i \sum_{[m,n] \in O_i} d[m,n]}{\sum_i |O_i|}$$

the mean grey level of pixels in image $Im_2$ is computed from said subset of pixels defined in image $Im_2$ and is referred to pixels defined in $Im_1$:

$$m_d = \frac{\sum_{[m,n]\in A} d[m,n]}{|A|}$$

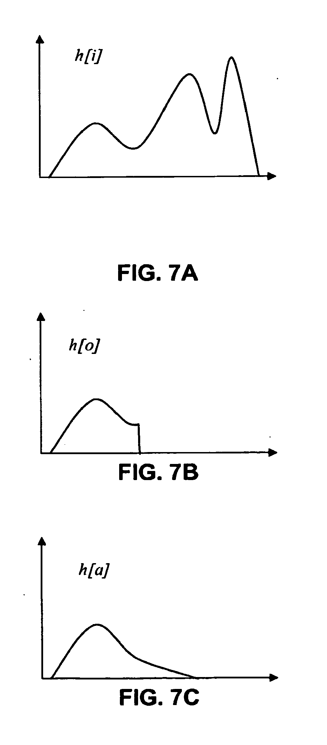

Other grey level statistics can be computed similarly: variance, standard deviation, skewness, krutosis, entropy... The benefit of the method is to define the pixels that are included in the calculation independently from their grey level, avoiding the disturbing effect of abrupt discontinuities in the probability function thereof. Referring to **FIG. 7**, $p[i]$, $p[a]$ and $p[o]$ be the probability functions of pixels from $Im_2$, $A$ and $O_i$ respectively, and $h[i]$, $h[a]$ and $h[o]$ represent the histograms thereof.

The extent of the labeled area varies according to the concentration of the primary antibody used in the present method. That is, the more concentrated the primary antibody, the larger the detected surface. In accordance with the present invention, the same primary antibody may be used in the first and the second immunomarking step. However, in such case, the concentration of primary antibody used in the second immunomarking step will be greater than the concentration used in the first immunomarking step. Thus a same primary antibody can be used for both density labeling and *reference area* labeling, provided that the immunohistochemical which produces the signal for densitometry measurements, is carried out with a concentration of the primary antibody at which variations of antigen content will result in proportional variations of optical density, whereas for the immunohistochemical procedure which produces the signal for reference area measurements, the same primary antibody is used at a concentration, higher than the previous one, that allows a maximum detection of reference area without however containing reliable information on density. The advantage of performing both immunomarking on the same antigen, is that the reference area is defined more precisely, resulting in more accurate density measurements, which are important when comparing information obtained from different samples.

For a definition of the term *"immunohistochemical system"*, reference is made to the method section above. In a preferred embodiment, an immunohistochemical system according to the invention is a system comprising at least three components, which are able to interact with one another. Said components comprise at least one antibody directed against an antigen of interest, a detection system provided with an enzyme and a chromogenic reagent, the latter being the substrate for the enzyme. It shall be clear that said at least three components of the system can be added separately, simultaneously or sequentially, when applied in the methods of the present invention.

The elution product X is to be understood as any solvent capable of eluting a chromogenic compound that precipitates in water. Examples of suitable elution product X include alcohols or hydrophobic solvents such as e.g. toluol or its derivatives.

The stabile chromogenic product is insoluble in further described elution solutions, while the labile chromogenic product is insoluble in reaction buffers but is soluble in elution solutions.

The term *"antigen"* as used herein refers to a substance that stimulates an immune response, especially the production of antibodies. Antigens usually are proteins or polysaccharides, but can be any type of molecule, including nucleic acid probes used to detect a specific DNA or RNA sequence.

The term *"reference antigen"* as used herein refers to antigens as defined herein which are useful targets to identify a reference area. In certain embodiments of the present invention, the reference antigen and the antigen of interest may be the same antigen.

In a preferred embodiment, the antibody-chromogen system I comprises a primary antibody AbI directed against said antigen, a detection system I able to detect said primary antibody AbI, and a chromogenic reagent I suitable for being converted by said detection system I into a stabile chromogenic product.

In another preferred embodiment, the antibody-chromogen system II comprises a primary antibody AbII directed against a reference antigen, a detection system II able to detect said primary antibody AbII, and a chromogenic reagent II suitable for being converted by said detection system into a labile chromogenic product.

The term *"primary antibody"* as used herein refers to an antibody, which is directed against an antigen, which may be an antigen present in a sample, herein also referred to as an antigen of interest, or which may be a reference antigen, as defined herein.

One elution product, elution product X, is suitable for eluting the labile chromogenic product. Said elution product X preferably is a solvent selected from the group comprising alcohols, and preferably is absolute ethanol. The solvent is capable of removing the labile chromogenic product while not altering the signal provided by the stabile chromogenic product. Another elution product, elution product Y, is suitable for eluting primary antibody AbI from the tissues before applying primary antibody AbII. Said elution product Y preferably is an aqueous acidic solution, preferably a HCl solution, and for instance a 0.1 N HCl solution in water

The primary antibody AbI is preferably directed against an antigen of interest selected from the group comprising:

- hormone receptors such as but not limited to estrogens, progesterone, androgens, growth hormone, insulin, IGF-I, IGF-II , somatostatin;
- membrane receptors for various endogeneous or exogeneous ligands such as but not limited to cRET, EGF, CD's, CEA, cKIT, cErbB2, VEGF, Ig's;
- specific vascular markers such as but not limited to CD31, CD34, VEGF-R subtypes, D2-40;
- specific solute transporters such as but not limited to NHE's, GLUT's, NCX's, NKCC's, CIC's,NBC's, AE's, ABC-family transporters, ion-translocating ATPases;
- membrane antigens of diagnostic or prognostic significance such as but not limited to CEA and its derivatives, various mucins including MUC family;
- proteins which are directly or inderectly involved in cell-to-cell or cell-to-ECM binding such as but not limited to laminin, tenascin, cadherins, catenins, intergrins, various CAM's;
- proteins of which the amount is correlated with differentiation or de-differentiation such as but not limited to p504s, PSA, cytokeratins...
- The above-indicated examples of antigens are potent targets for density measurement for scientific or clinical purposes.

The primary antibody AbII is preferably directed against a reference antigen selected from the group comprising:

- cytokeratins which usually label intensely carcinoma cells such as but not limited to CK22, CK7, AE1/AE3;
- Vimentin which stains the cytoplasm of mesenchymal cells;
- Glial Fibrillary Acidic Protein which labels the cells in the central nervous system;
- Lamins which are found in the nuclear membrane of most cells;
- hormones such as but not limited to insulin, glucagon, somatostatin, gastrin, cholecystokinin, growth hormone, prolactin, different forms of HCG, POMC, serotonin;
- Organelle-specific proteins such as but not limited to synaptoptiysin, chromogranin;
- Tissue or state-related enzymes, such as but not limited to thyrosin-hydroxylase, cytochrome P450 enzymes, PSA, PAP, GSTP. The term "state-related" refers to enzymes that are expressed in a particular metabolic or developmental state. for example, following induction by pharmacologic agents, some isoforms of the cytochrome P450 will be detectable in liver cells whereas under other conditions, they are not.

The immunohistochemical (antibody-chromogen) systems for use in the present invention thus preferably comprise at least a primary antibody directed against the antigen, a detection system provided with an enzyme and a chromogenic reagent, the latter being the substrate for the enzyme

A further component included for use in the present invention can be a secondary antibody recognizing the primary antibody. The term *"secondary antibody"* as used herein refers to an antibody, which is directed against a primary antibody, as defined herein. The secondary antibody can be coupled directly or indirectly with an enzyme. Another possibility is that the secondary antibody is conjugated to a coupling molecule. The term *"coupling molecule"* as used herein refers to any type of molecule which is capable of forming a conjugate with an antibody, and preferably with a secondary antibody (or tertiary antibody - see below) as defined herein. The coupling molecule can be recognized by a complementing molecule that is bound to an enzyme. The term *"complementing molecule"* as used herein refers to a molecule which is capable of recognizing a coupling molecule as defined herein. Alternatively the coupling molecule can be provided with an enzyme. In an non-limitative example, the coupling molecule is biotin, which is subsequently detected by a complementing molecule, such as for example (strept)avidin, the latter being bound to an enzyme. In another example the coupling molecule is a macromolecule as defined herein which is conjugated to an enzyme (see below).

It is also contemplated in the present invention that a secondary antibody can be recognized by another antibody, e.g. a tertiary antibody. The term *"tertiary antibody"* as used herein refers to an antibody, which is directed against a secondary antibody, as defined herein. A tertiary antibody can be but need not be coupled to a coupling molecule, which in turn is recognized by another (complementing) molecule or antibody. It shall be understood from the present invention that a tertiary antibody may be directly or indirectly coupled with an enzyme in the same way as was described for a secondary antibody.

It shall be understood that the detection systems for use in the present invention can thus provide detection in one or multiple steps, e.g. when applying antibodies (for instance secondary antibodies) conjugated to a coupling molecule, or when using a cascade of antibodies (e.g. secondary, tertiary, quaternary, etc... antibodies). Use of a cascade of antibodies advantageously allows amplification of the detection signal.

As is clear to the person skilled in the art, there are many different possibilities to detect an antigen, and the ones mentioned here are strictly illustrative and don't restrict the method of the invention in any way to the examples described.

In one embodiment, the detection system I for use in the invention comprises a secondary antibody that is conjugated to a coupling molecule, as defined herein, whereby said secondary antibody is directed against said primary antibody AbI, and a complementing molecule, able to recognize the coupling molecule, and being conjugated to an enzyme. In another embodiment, the detection system II for use in the invention comprises a secondary antibody that is conjugated to a coupling molecule, as defined herein, whereby said secondary antibody is directed against said primary antibody AbII, and a complementing molecule, able to recognize the coupling molecule, and being conjugated to an enzyme. In one preferred embodiment, said detection system I comprises a secondary antibody that is conjugated to biotin, whereby said secondary antibody is directed against said primary antibody AbI, and an avidin- or streptavidin-conjugated enzyme. In another preferred embodiment, said detection system II comprises a secondary antibody that is conjugated to biotin and directed against said primary antibody AbII and an avidin- or streptavidin-conjugated enzyme.

In another preferred embodiment, said detection system I comprises a secondary antibody directed against said primary antibody AbI, whereby said secondary antibody is bound to a macromolecule-conjugated enzyme. In yet another embodiment, said detection system II comprises a secondary antibody directed against said primary antibody AbII, and whereby said secondary antibody is bound to a macromolecule-conjugated enzyme. The term *"macromolecule"* is to be understood herein as to refer to a polymer and molecules, which structurally include polymers. Said macromolecule may for instance be a polysaccharide, e.g. dextran, on which secondary antibodies and (revelation) enzymes, e.g. peroxidase or alkaline phosphatase, may be bound. The term "macromolecule" is well known by a person of skill in the present field of immunohistochemistry.

In another embodiment, said detection system I comprises A) a secondary antibody directed against said primary antibody AbI, whereby said secondary antibody is optionally conjugated to a coupling molecule and wherein a complementing molecule, able to recognize the coupling molecule, and being conjugated to an enzyme, is further provided, and B) a tertiary antibody-enzyme conjugate, whereby said conjugate comprises a tertiary antibody that is directed against said secondary antibody said detection system I comprises A) a secondary antibody directed against said primary antibody AbI, whereby said secondary antibody is optionally bound to a macromolecule-conjugated enzyme, and B) a tertiary antibody-enzyme conjugate, whereby said conjugate comprises a tertiary antibody that is directed against said secondary antibody.

In another embodiment, said detection system II comprises A) a secondary antibody directed against said primary antibody AbII, whereby said secondary antibody is optionally conjugated to a coupling molecule and wherein a complementing molecule, able to recognize the coupling molecule, and being conjugated to an enzyme, is further provided, and B) a tertiary antibody-enzyme conjugate, whereby said conjugate comprises a tertiary antibody that is directed against said secondary antibody. Said detection system II comprises A) a secondary antibody directed against said primary antibody AbII, whereby said secondary antibody is optionally bound to a macromolecule-conjugated enzyme, and B) a tertiary antibody-enzyme conjugate, whereby said conjugate comprises a tertiary antibody that is directed against said secondary antibody.

In a preferred embodiment, said detection system I comprises: A) a secondary antibody which is directed against said primary antibody AbI, and whereby said secondary antibody is optionally conjugated to biotin, and wherein an avidin- or streptavidin-conjugated enzyme is further provided in the system, and B) a tertiary antibody-enzyme conjugate, comprising a tertiary antibody which is directed against said secondary antibody. In yet another preferred embodiment, said detection system I comprises: A) a secondary antibody which is directed against said primary antibody AbI, and whereby said secondary antibody is optionally bound to a dextran-conjugated enzyme, and B) a tertiary antibody-enzyme conjugate, comprising a tertiary antibody which is directed against said secondary antibody.

In another preferred embodiment, said detection system II comprises: A) a secondary antibody which is directed against said primary antibody AbII, and whereby said secondary antibody is optionally conjugated to biotin, and wherein an avidin- or streptavidin-conjugated enzyme is further provided in the system, and B) a tertiary antibody-enzyme conjugate, comprising a tertiary antibody which is directed against said secondary antibody. In yet another preferred embodiment, said detection system II comprises: A) a secondary antibody which is directed against said primary antibody AbII, and whereby said secondary antibody is optionally bound to a dextran-conjugated enzyme, and B) a tertiary antibody-enzyme conjugate, comprising a tertiary antibody which is directed against said secondary antibody.

In a further preferred embodiment, the enzyme which is coupled to the complementing molecule, or the enzyme which is coupled to the macromolecule -when present- may be the same as or different from the enzyme in the tertiary antibody-enzyme conjugate. In a preferred embodiment, the enzyme conjugated to avidin- or streptavidin- or the enzyme conjugated to a macromolecule (e.g. dextran) may be the same as or different from the enzyme in the tertiary antibody-enzyme conjugate.

In another embodiment, said detection system I comprises:

a secondary antibody directed against said primary antibody AbI whereby said secondary antibody is conjugated to a reporter, and
an antibody directed against said reporter, whereby said antibody is conjugated to an enzyme or whereby said antibody is bound to a macromolecule-conjugated enzyme.

In yet another embodiment, said detection system II comprises:

a secondary antibody directed against said primary antibody AbII whereby said secondary antibody is conjugated to a reporter, and

an antibody directed against said reporter, whereby said antibody is conjugated to an enzyme or whereby said antibody is bound to a macromolecule-conjugated enzyme.

The term *"reporter"* as used herein refers to any type of molecule which is suitable for being coupled or conjugated to a secondary antibody. In certain embodiments, the term "reporter" and "coupling molecule", as used herein, may be considered to be synonyms. In a preferred embodiment, the reporter is FITC, digoxigenin, or biotin. The reporter preferably serves as secondary antigen on the tail of a secondary antibody to make the latter recognizable by a tertiary antibody, whereby said tertiary antibody may be labeled in a similar way as secondary antibodies. Such approach allows further amplification of the signal when needed. In the case of FITC as reporter, FITC is not used here for its fluorescent properties.

According to the present invention, the enzymes may comprise any type of enzymes which are capable of reacting with a chromogenic reagent. Preferably in the above-indicated methods the enzyme is a peroxidase or an alkaline phosphatase.

According to the present invention, the chromogenic reagents I may comprise any type of reagents which are capable of forming a stabile chromogenic product. In a preferred embodiment, the chromogenic reagent I of antibody-chromogen system I is selected from the group comprising diaminobenzidine (DAB), and NewFuchsin. In an example, the chromogenic reagent diaminobezidine (DAB) is used in combination with peroxidase.

Further according to the present invention, the chromogenic reagents II may comprise any type of reagents which are capable of forming a labile chromogenic product. In a preferred embodiment, the chromogenic reagent II of antibody-chromogen system II is a chromogenic reagent which forms a precipitate after reaction that can be subsequently solubilized. Preferably the chromogenic reagent II forms an alcohol-soluble chromogenic precipitate. Preferably the chromogenic reagent II of antibody-chromogen system II is selected from the group comprising NBT, Fast-red, Fast-blue, amino-ethylcarbazole. In an example chromogenic reagents II used in combination with alkaline phosphatase preferably include Fast-Red or Fast-Blue, whereas amino-ethylcarbazole is a chromogenic reagent II suitable for use in conjunction with peroxidase.

In conclusion, quantitative evaluation of immunohistochemical labeling has been performed for more than 30 years. Surprisingly, unlike other quantitative methods such as quantitative PCR, that type of evaluation methods is not widely used, possibly because of the difficulty to determined a reference. Immunohistochemical analyses have to compromise with many variables, which can be difficult to standardize. Some of them are independent of the labeling itself. They include tissue processing, fixation, embedding, and thickness of the sections or, possibly, the storage thereof. Some factors directly depend on the labeling, including characteristics, concentration, dilution buffer, of primary antisera and chromogene. Moreover, the use of digital images as a medium to quantify the resulting signal brings up additional parameters. Consequently, the reliability of measurements carried possibly through digital images of an immunohistochemical signal, depends on proper conditions of detection and acquisition. Especially, an accurate definition of the reference area is a matter of importance that can considerably reduce some aspects of the variability recorded in immunohistochemical studies. The present invention demonstrates amongst other things the importance of an accurate detection of the reference area in sensitive immunohistodensitometric measurements.

The following examples are intended to illustrate particular embodiments of the invention, and are not intended to limit the scope of the invention.

## EXAMPLES

*Example 1: Determining antigen content in a sample using double immunohistochemical detection*

In this example, a double immunohistochemical technique is used to evaluate the impact of reference area determination on a model of rats treated with glibenclamide to elicit a reduction of insulin content, and a chronological series of rats to study the ontogeny of GLUT2 in the islets of Langerhans. A first immunohistochemical procedure is carried out in conditions such that the density of the immunostaining can be evaluated, whereas, on the same slide, a similar procedure using a saturated antibody is applied to establish the reference area.

Material and methods

***Animal tissues***

Wistar rats (230 - 280 g) were injected twice at 12 hour interval with glibenclamide at 5.0 mg/kg body weight (n=3) to induce insulin release and reduce its content in the islets of Langerhans or with saline (n=3).

In addition, male control Wistar rats were divided in five categories (n=5 each category) grouped for age as follows: fetuses (day 20), newborn (day 1), weaned (day 27), young (4 month) and aged (15 month). Animals were fed *ad libitum* and killed by decapitation.

### *Immunohistochemistry*

Paraffin-embedded pancreases were cut to five-micrometer sections. Slides were dewaxed and rehydrated in a graded series of ethanols. Endogenous peroxidase activity was blocked by 0.3% hydrogen peroxide for 30 min at room temperature. Nonspecific antibody staining was blocked by pre-incubation with 10% normal goat serum in Tris-HCl (0.05M, pH 7.4) for 30 min at room temperature. The slides were incubated overnight at 4°C with mouse anti-insulin antibody (or mouse anti-GLUT2 antibody) diluted at 1/1000 or 1/2000 in Tris containing 1% bovine serum albumin (BSA). After washes in Tris, slides were incubated successively with biotinylated anti-mouse antibody and then with streptavidin-peroxidase conjugate, both at 1/500 for 30 min at room temperature. The peroxidase activity was revealed by incubating with 3,3'-diaminobenzidine hypochloride (DAB - 50mg/100mL supplemented with $H_2O_2$ 0.02%) for 10 min which produced a brown deposit.

A second immunolabeling was performed using the anti-insulin antibody (or mouse anti-GLUT2 antibody) at a dilution such that the stained area corresponded to the actual area independently of the amount of antigen contained in the cells. To do so, after being rinsed in tap water, the slides were treated with 0.1M hydrochloric acid for 30 min to elute previously bound antibodies, followed by incubation with 10% normal goat serum in Tris pH 7.4 for 30 min at room temperature. Mouse anti-insulin antibody (1/500 in Tris pH 7.4 containing 1% BSA) was applied overnight at 4°C. After rinsing, the sections were incubated with rabbit anti-mouse antiserum (1/70) and then with mouse alkaline phosphatase - anti-alkaline phosphatase system (1/50). The alkaline phosphatase activity was revealed by immersion in freshly prepared solution containing FastRed (FR - 500 mg/L), levamisole (400 mg/L) and naphtol (500 mg/L) in Tris buffer (0.05M, pH 9.0), yielding a double immunostaining. Slides were mounted with a glycerin solution. All the slides were processed simultaneously to allow comparative optical density measurements between samples. After digitalisation, the FastRed deposit was removed from the slide by immersion in methanol. The slides were then mounted in Eukitt medium (Eukitt, Freiburg, Germany).

### *Quantification: Sequential measurement of a double immunolabeling*

Sections were analysed through a Zeiss microscope equipped with a scanning stage. Illumination was produced by a stabilized light source and a continuous interference filter monochromator with a 14-nm bandwidth. Fields were captured in two steps through a 16X objective by a Dage-MTI CCD camera into 752 X 560 8bits depth pixels (0.456 X 0.476 μm/pixel). Digital image handling and measurement processes were developed as macros under KS-400 image analysis system.

The islets on each slide bearing the double immunostaining were first digitized whereas spatial coordinates of the stage were kept in a file. After dissolution of the FR precipitate, islet positions were rather well recovered. The procedure allowed the operator to match position exactly by superimposition of the digitized and the online image before capturing. This co-registration process generated two strictly super-imposable images of the same islet: one with a double immunostaining (DI : FR+DAB) used to define the reference area and one with the single immunostaining (SI : DAB alone) dedicated to optical density measurement.

Two absorbance values were computed following two definitions of the reference area.

The two definitions of the reference area are related to the definitions of the two subsets of pixels either labeled by the stabile chromogenic product or by the labile chromogenic product. In the first case, the mean grey level necessary to computed SOD is given by:

$$m_o = \frac{\sum_i \sum_{[m,n] \in O_i} d[m,n]}{\sum_i |O_i|}$$

Whereas in the second case, it is given by:

$$m_d = \frac{\sum_{(m,n)\in A} d\{m,n\}}{|A|}$$

Similarly, 'direct absorbance' refers to the former and 'referred absorbance' refers to the latter.

The 'direct absorbance' was measured on the SI image, and therefore took only the DAB stained pixels into account. The 'referred absorbance' was computed by dividing the sum of the grey level of each stained pixel in the SI image, by the number of pixels (i.e. the area) of the reference area, as delimited on the DI image. The grey level of the background was measured in an adjacent area of unstained tissue, and used to calculate the specific absorbance of the labeling.

*Results*

The use of an anti-insulin antibody at a dilution of 1/2000 or 1/1000 labeled a insular β cell area corresponding respectively to 95% or 75% of the area detected by an anti-insulin antibody at a dilution of 1/500 in control rats, and of 58% or 38% in glibenclamide-treated rats.

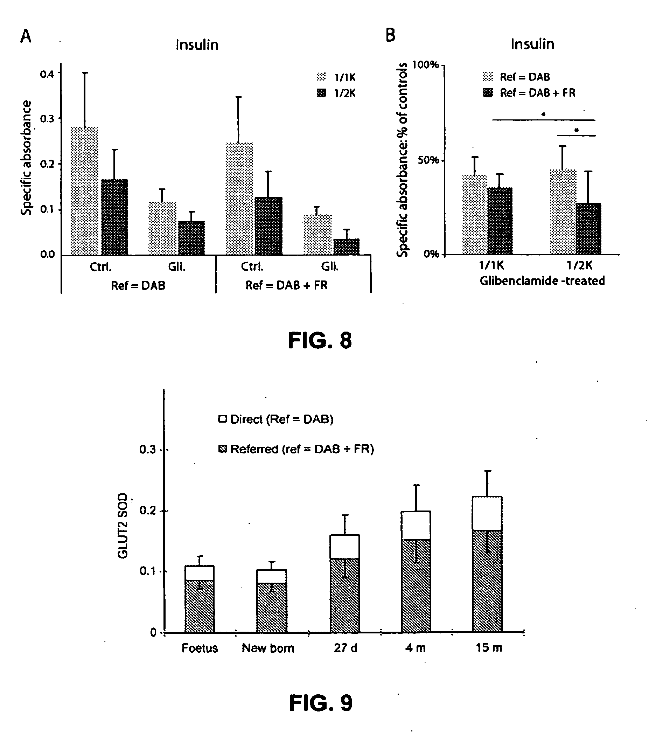

The absorbance of the insulin immunostaining was affected by islet insulin content, primary antibody dilution, and the chosen reference area. The reduction of insulin content by glibenclamide treatment resulted in a reduction of the specific optical density of insulin immunostaining. Similarly, the dilution of the primary antibody resulted in a reduction of the specific optical density, but this diminution was less marked when the antigen content was low (glibenclamide, **Fig. 8A**). The amplitude of reduction of the insulin immunohistochemical density in response to a diminution of the antigen content (glibenclamide) or to a dilution of the primary antibody is influenced by variations secondary to the different modes of determining the reference area (**Fig. 8B**). When the β cell reference area was determined carried on to detected even poorly granulated β cells, using high anti-insulin antibody concentration in a second immunostaining, dilution of the primary antibody resulted in a further reduction of insulin absorbance even though the antigen content was already low due to glibenclamide treatment ($p < 0.05$).

The influence of the reference area on GLUT2 expression throughout life was studied similarly and the results are depicted in **Fig. 9.** The ratio between the area of GLUT2 labeling and the total β-cell surface identified by insulin immunostaining, was 10% in foetuses, 13% in new born and 21-22% in older animals. The intensity of GLUT2 immunostaining progressed with the age of the animals. The referred absorbance was lower than the direct absorbance in every age group. The differences between direct and referred absorbance were less marked in foetus and newborn group, although it did not modify the overall trend of progression with age.

*Discussion*

The intensity of immunostaining depends on both the antigen content and the dilution of the antibody. Both parameters also interfere with the detected area, the diminution of which may be compensated by increasing the concentration of the primary antibody.

In this study, variations in insulin immunostaining intensity were induced by reduction of insulin content, by a pharmacological agent, or by dilution of the primary antibody. The primary antibody concentration was chosen to allow detection of antigen content variation. Under these conditions, low staining intensities corresponded to smaller labeling areas, which resulted in overestimated SOD value.

On the experimental material, SOD variations related to the reduction of insulin immunostaining intensity were less marked when the reference area for absorbance measurements was given by the immunohistochemical signal itself, than when the reference area was improved by a second immunolabeling to fit the complete profile of the β cells cytoplasm. Using a more accurate determination of the reference area statistically increased the SOD difference observed between experimental groups.

The evaluation of the density of a membrane staining entails a similar issue. The SOD along the membrane is likely to represent the density of antigen thereof. To evaluate the amount of antigen per cell, this measure should be corrected for cell volume. The case study was that of GLUT2 ontogeny. The double immunostaining method was applied to evaluate GLUT2 expression in pancreatic β cells at different ages where variations of cell volume could interfere with the results. Measurements of GLUT2 expression increased with age, regardless whether they were computed using the membrane or the whole cell as reference area. As expected, values were lower with the latter method than with the former. However, the difference between referred and direct densities was lower in foetus and newborn than in other groups. Between groups, the cell volume was evaluated through measurement of the number of nuclei per area of section. It indicated a variation in cell volume, which, although slight in intensity, corresponds to differences in SOD recorded when comparing the usual method with that provided by the invention.

*Example 2*: *Determination of insulin (antigen) specific optical density by three different methods*

In the present example, it was known that by using a pharmacological agent a dramatic reduction (70%) of antigen content (insulin) can be achieved.

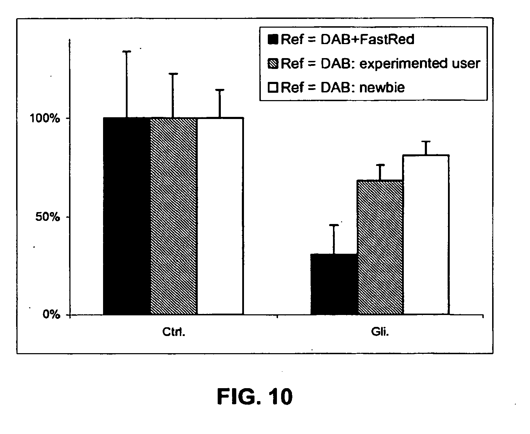

Antigen content of samples were determined using three different approaches: A) single staining procedure whereby the operator is not aware of the possible caveats; B) single staining procedure whereby the operator is aware of the possible caveats; B) double immunostaining procedure according to the present invention using a same primary antibody for the antibody-chromogen systems I and II and using DAB and FastRed® as chromogenic reagents. Results of the experiment are shown in **FIG. 10.**

The reduction of the labeled area in comparison to the actual reference area resulted in alteration of density measurements when they were carried out with a single immunostaining. When the operator/observer was not aware of the possible caveats (DAB-newbie), density measurements were not different from one situation to the other (only 81% reduction). When the operator/observer was aware of possible issues (DAB-experimented user), he tries to correct the measurements (68% reduction), but the amount of reduction which is computed is far from that expected upon observation. Using a supplementary immunostaining procedure (DAB+FastRed) wherein with the same primary antibody is used at a higher concentration than that used for density measurements, which then allowed a complete covering of the reference area with a removable chromogen, permitted to compute the expected 70% reduction of antigen content.

*Example 3: examples of primary antibodies AbI and AbII useful in the present method and kit.*

Sets of primary antibodies AbI and AbII which can be applied in the present method and kit are hereby illustrated:

- For an insulin quantitation kit: Ab I : anti insulin 1/2000 (Chemicon); Ab II : anti-insulin 1/500 (Chemicon).
- for an insular GLUT-2 quantitation kit: Ab I : anti-GLUT-2 1/2000 (Chemicon); Ab II : anti-insulin 1/500 (Chemicon),
- for a liver GLUT-2 quantitation kit: Ab I : anti-GLUT-2 1/2000 (Chemicon); Ab II : anti-CK22 1/100 (BioMeda),
- for a GTSP in prostate cancer cells quantitation kit: Ab I : anti-GSTP; Ab II: anti-p504s,
- for a cERB B2 in epithelial cells quantitation kit: Ab I : anti-cERB-B2, Ab II : anti-CK22,
- for a pro-insulin mRNA in pancreatic B cells quantitation kit: AbI : Digoxigenin-labeled pro-insulin mRNA hybridization probe and anti-Digoxigenin antibody; Ab II : Anti-IAPP

*Example 4: Examples of detection systems and elution products useful in the present method and kit.*

Sets of examples of detection systems and elution products useful in the present method and kit which can be applied in the present method and kit are hereby illustrated:

Set 1:

Anti-mouse Fab'
Anti-rabbit Fab' biotin-conjugate
Streptavidin-peroxidase
Mouse alkalin phosphatase - anti-mouse-alkalin phospatase system FastRed/Naphtol AS-MX
3'-3'-diaminobenzidine
0.1N HCl
Absolute ethanol 0.05M Tris-HCl pH 7.4

Set 2:

Anti-mouse Fab' biotin-conjugate
Anti-rabbit Fab'
Streptavidin-peroxidase
Mouse alkalin phosphatase - anti-mouse-alkalin phospatase system
FastRed/Naphtol AS-MX
3'-3'-diaminobenzidine
0.1N HCl
Absolute ethanol
0.05M Tris-HCl pH 7.4

Set 3:

Anti-mouse En-Vision - POD
En-Vision - AP
FastRed/Naphtol AS-MX
3'-3'-diaminobenzidine
0.1N HCl
Absolute ethanol
0.05M Tris-HCl pH 7.4

**Claims**

1. A method for determining antigen content in a region of interest of a sample comprising the steps of:

a) providing a slide containing said sample,
b) submitting said sample to immunomarking comprising the steps of:

b1) optionally submitting said sample to a stabile immunomarking by incubating said sample with an antibody-chromogen system I thereby forming a stabile chromogenic product not soluble in buffering solutions and mounting media, and
b2) submitting said sample to a labile immunomarking by incubating said sample with an antibody-chromogen system II thereby forming a labile chromogenic product which precipitates under aqueous conditions and which can be solubilized in alcohols,

c) acquiring first image(s) of said sample containing said labile chromogenic product optionally in combination with said stabile chromogenic product, thereby defining a reference area,
d) eluting said labile chromogenic product using an elution product selected from the group consisting of alcohols or hydrophobic solvents such as toluol,
e) provided that step b1) was not carried out, submitting the sample of step d) to a stabile immunomarking by incubating said sample with an antibody-chromogen system I thereby forming a stabile chromogenic product;
f) acquiring second image(s) of said sample containing said stabile chromogenic product,
g) processing said first and second image(s) by superimposing the first image(s) on the second image(s) or by superimposing a mask extracted from said first image(s) on the second image(s), and
h) determining antigen content of said sample within the defined reference area.

2. Method according to claim 1, wherein incubation of said sample with an antibody-chromogen system I, said antibody-chromogen system I comprising a primary antibody AbI directed against said antigen, a detection system I able to detect said primary antibody AbI, and a chromogenic reagent I suitable for being converted by said detection system I into a stabile chromogenic product, said incubation comprising the subsequent steps of:

- incubating the sample with the primary antibody AbI of the antibody-chromogen system I
- adding said detection system I and said chromogenic reagent I to said incubated sample thereby forming a stabile chromogenic product, and
- optionally removing bound primary antibody AbI by acidic elution.

3. Method according to any of claims 1 or 2, wherein incubation of said sample with an antibody-chromogen system II, said antibody-chromogen system II comprising a primary antibody AbII directed against said antigen, a detection system II able to detect said primary antibody AbII, and a chromogenic reagent II suitable for being converted by said detection system II into a labile chromogenic product, said incubation comprising the subsequent steps of:

- incubating said sample with the primary antibody AbII of the antibody-chromogen system II, and
- adding said detection system II and said chromogenic reagent II to said incubated sample thereby forming a labile chromogenic product.

4. Method according to any of claims 1 to 3, wherein images are acquired by:

- mounting the slide containing the sample on a computer-controlled optical microscope equipped with a digital camera and a motorized stage,
- acquiring digital images of different areas of said sample, and
- storing said images on a suitable storage means.

5. Method according to any of claims 1 to 4, wherein said antigen content is determined by calculating optical density (SOD) of the superimposed image using an algorithm, wherein

Im corresponds to the compound image of $Im_1$ and $Im_2$

$Im_1$ corresponds to an image with a labile and optionally also a stabile chromogenic product,

$Im_2$ corresponds to an image with a labile chromogenic product,

A corresponds to a region covered by the labile immunomarking,

$O_i$ corresponds to a region covered by the stabile immunomarking, and

let $a[m,n] \in A$, $o[m,n] \in O_i$ be pixels of image Im,

and $d[m,n]$, be the grey level of pixel $a[m,n]$ in image $Im_2$

then surface density of the labile immunomarking in the stabile immunomarking is given by:

$$\frac{|A|}{\sum |O_i|}$$

the mean grey level of a subset of pixels defined in image $Im_2$ is defined by :

$$m_o = \frac{\sum_i \sum_{[m,n]\in O_i} d[m,n]}{\sum_i |O_i|}$$

the mean grey level of pixels in image $Im_2$ is computed from said subset of pixels defined in image $Im_2$ and is referred to pixels defined in $Im_1$:

$$m_d = \frac{\sum_{[m,n]\in A} d[m,n]}{|A|} \ .$$

**Patentansprüche**

1. Verfahren zum Bestimmen des Antigengehalts in einem interessierenden Bereich einer Probe, umfassend die Schritte:

a) Bereitstellen eines Objektträgers, der die Probe beinhaltet,
b) Probe einer Immunmarkierung unterziehen, welche die folgenden Schritte umfasst:

b1) gegebenenfalls die Probe einer stabilen Immunmarkierung durch Inkubieren der Probe mit einem Antikörper-Chromogen-System I unterziehen, wodurch ein stabiles chromogenes Produkt gebildet wird, das in puffernden Lösungen und Einbettungsmedien nicht löslich ist; und
b2) die Probe einer labilen Immunmarkierung durch
Inkubieren der Probe mit einem Antikörper-Chromogen-System II unterziehen, wodurch ein labiles chromogenes Produkt gebildet wird, das unter wässrigen Bedingungen ausfällt und in Alkoholen gelöst werden kann,

c) Erstellen von einer ersten Abbildung / ersten Abbildungen von der Probe, die das labile chromogene Produkt enthalten, gegebenenfalls in Kombination mit dem stabilen chromogenen Produkt, wodurch ein Referenzbereich definiert wird.

d) Eluieren des labilen chromogenen Produkts unter Verwendung eines Elutionsproduktes, das ausgewählt ist aus der Gruppe, die aus Alkoholen oder hydrophoben Lösemitteln wie beispielsweise Toluol besteht.

e) sofern Schritt b1) nicht durchgeführt wurde, Probe von Schritt d) durch Inkubieren der Probe mit einem Antikörper-Chromogen-System I einer stabilen Immunmarkierung unterziehen, wodurch ein stabiles chromogenes Produkt gebildet wird;

f) Erstellen von einer zweiten Abbildung / zweiten Abbildungen der Probe, die das stabile chromogene Produkt enthält.

g) Bearbeiten der ersten und zweiten Abbildung / der ersten und zweiten Abbildungen durch Überlagern der ersten Abbildung / der ersten Abbildungen über die zweite Abbildung / die zweiten Abbildungen oder durch Überlagern einer Maske, die aus der ersten Abbildung / den ersten Abbildungen extrahiert wurde, über die zweite Abbildung / die zweiten Abbildungen, und

h) Bestimmen des Antigengehalts der Probe innerhalb von dem definierten Referenzbereich.

2. Verfahren nach Anspruch 1, wobei die Probe mit einem Antikörper-Chromogen-System I inkubiert wird, das Antikörper-Chromogen-System I einen primären Antikörper AbI, der gegen das Antigen gerichtet ist, ein Nachweissystem I, das imstande ist, den primären Antikörper AbI nachzuweisen, und ein chromogenes Reagenz I umfasst, das für die Umwandlung in ein stabiles chromogenes Produkt durch das Antikörper-Chromogen-System I geeignet ist, die Inkubation die nachfolgenden Schritte umfasst:

   - Inkubieren der Probe mit dem primären Antikörper AbI aus dem Antikörper-Chromogen-System I
   - Hinzufügen von Nachweissystem I und dem chromogenen Reagenz I zu der inkubierten Probe, wodurch sich ein stabiles chromogenes Produkt bildet, und
   - gegebenenfalls Entfernen des gebundenen primären Antikörpers AbI durch saure Elution.

3. Verfahren nach einem der Ansprüche 1 oder 2, wobei die Probe mit einem Antikörper-Chromogen-System II inkubiert wird, das Antikörper-Chromogen-System II einen primären Antikörper AbII, der gegen das Antigen gerichtet ist, ein Nachweissystem II, das imstande ist, den primären Antikörper AbII nachzuweisen, und ein chromogenes Reagenz II umfasst, das für die Umwandlung in ein stabiles chromogenes Produkt durch das Antikörper-Chromogen-System II geeignet ist, die Inkubation die nachfolgenden Schritte umfasst:

   - Inkubieren der Probe mit dem primären Antikörper AbII aus dem Antikörper-Chromogen-System II, und
   - Hinzufügen von Nachweissystem II und dem chromogenen Reagenz II zu der inkubierten Probe, wodurch sich ein labiles chromogenes Produkt bildet.

4. Verfahren nach einem der Ansprüche 1 bis 3, wobei die Abbildungen erstellt werden durch:

   - Befestigen des Objektträgers, der die Probe enthält, in einem computergesteuerten optischen Mikroskop, das mit einer Digitalkamera und einem motorisierten Tisch ausgerüstet ist,
   - Erstellen digitaler Abbildungen von verschiedenen Bereichen der Probe, und
   - Speichern der Abbildungen auf einem geeigneten Speichermittel.

5. Verfahren nach einem der Ansprüche 1 bis 4, wobei der Antigengehalt über die Berechnung der spezifischen optischen Dichte (SOD) von der überlagerten Abbildung unter Verwendung von einem Algorithmus bestimmt wird, wobei

   Im der Verbindungs-Abbildung von $Im_1$ und $Im_2$ entspricht

   $Im_1$ der Abbildung mit einem labilen und gegebenenfalls auch einem stabilen chromogenen Produkt entspricht,

   $Im_2$ der Abbildung mit einem labilen chromogenen Produkt entspricht,

   A einem Bereich entspricht, der durch die labile Immunmarkierung bedeckt ist,

   $O_i$ einem Bereich entspricht, der durch die stabile Immunmarkierung bedeckt ist, und

   wenn $a[m,n] \in A$, $o[m,n] \in O_i$ die Pixel von Abbildung Im sind,

   und d [m, n] der Grauwert von Pixel a [m, n] in Abbildung $Im_2$ ist;

   dann ist die Oberflächendichte der labilen Immunmarkierung bei der stabilen Immunmarkierung gegeben durch:

$$\frac{|A|}{\sum |O_i|}$$

der mittlere Grauwert von einer Teilmenge der Pixel, die in Abbildung $Im_2$ festgelegt sind, ist definiert durch:

$$m_o = \frac{\sum_i \sum_{[m,n] \in O_i} d[m,n]}{\sum_i |O_i|}$$

und der mittlere Grauwert der Pixel in Abbildung $Im_2$ wird berechnet aus der Teilmenge der Pixel, die in Abbildung $Im_2$ definiert sind, und bezogen auf die Pixel, die in $Im_1$ definiert sind:

$$m_d = \frac{\sum_{[m,n] \in A} d[m,n]}{|A|}$$

**Revendications**

1.  Procédé de détermination du contenu antigénique dans une région d'intérêt d'un échantillon qui comprend les étapes consistant à :

    a) fournir une lamelle contenant ledit échantillon,
    b) soumettre ledit échantillon à un marquage immunologique qui comprend les étapes consistant à :

    b1) facultativement soumettre ledit échantillon à un immunomarquage stable par incubation dudit échantillon avec un système anticorps-chromogène I, formant ainsi un produit chromogène stable insoluble dans des solutions tampons et des milieux de montage, et
    b2) soumettre ledit échantillon à un immunomarquage labile par incubation dudit échantillon avec un système anticorps-chromogène II formant ainsi un produit chromogène labile qui précipite dans des conditions aqueuses et qui peut être solubilisé dans des alcools,

    c) acquérir une (des) première(s) image(s) dudit échantillon contenant ledit produit chromogène labile facultativement en combinaison avec ledit produit chromogène stable, définissant ainsi une zone de référence,
    d) éluer ledit produit chromogène labile en utilisant un produit d'élution choisi dans le groupe constitué par les alcools ou les solvants hydrophobes tels que le toluol,
    e) à condition que l'étape b1) n'ait pas été réalisée, soumettre l'échantillon de l'étape d) à un immunomarquage stable par incubation dudit échantillon avec un système anticorps-chromogène I formant ainsi un produit chromogène stable ;
    f) acquérir une (des) seconde(s) image(s) dudit échantillon contenant ledit produit chromogène stable,
    g) traiter lesdites première(s) et seconde(s) image(s) par superposition de la (des) première(s) image(s) sur la (les) seconde(s) image(s) ou par superposition d'un masque extrait de ladite (desdites) première(s) image(s) sur la (les) seconde(s) image (s), et
    h) déterminer le contenu antigénique dudit échantillon dans la zone de référence définie.

2.  Procédé selon la revendication 1, dans lequel l'incubation dudit échantillon avec un système anticorps-chromogène I, ledit système anticorps-chromogène I comprenant un anticorps primaire AbI dirigé contre ledit antigène, un système de détection I capable de détecter ledit anticorps primaire AbI, et un réactif chromogène I adapté pour être converti par ledit système de détection I en un produit chromogène stable,

ladite incubation comprenant les étapes subséquentes consistant à :

- incuber l'échantillon avec l'anticorps primaire AbI du système anticorps-chromogène I,
- ajouter ledit système de détection I et ledit réactif chromogène I au dit échantillon incubé formant ainsi un produit chromogène stable, et
- facultativement éliminer l'anticorps primaire AbI lié par élution acide.

3. Procédé selon l'une quelconque des revendications 1 ou 2, dans lequel l'incubation dudit échantillon avec un système anticorps-chromogène II,
ledit système anticorps-chromogène II comprenant un anticorps primaire AbII dirigé contre ledit antigène, un système de détection II capable de détecter ledit anticorps primaire AbII, et un réactif chromogène II adapté pour être converti par ledit système de détection II en un produit chromogène labile,
ladite incubation comprenant les étapes subséquentes consistant à :

- incuber ledit échantillon avec l'anticorps primaire AbII du système anticorps-chromogène II, et
- ajouter ledit système de détection II et ledit réactif chromogène II au dit échantillon incubé pour ainsi former un produit chromogène labile.

4. Procédé selon l'une quelconque des revendications 1 à 3, dans lequel les images sont acquises par :

- montage de la lamelle contenant l'échantillon sur un microscope optique commandé par ordinateur doté d'une caméra numérique et d'un étage motorisé,
- acquisition d'images numériques de différentes zones dudit échantillon, et
- stockage desdites images sur un support de stockage adapté.

5. Procédé selon l'une quelconque des revendications 1 à 4, dans lequel ledit contenu antigénique est déterminé par calcul de la densité optique (SOD) de l'image superposée en utilisant un algorithme,
où
Im correspond à l'image composée de Im$_1$ et Im$_2$,
Im$_1$ correspond à une image avec un produit chromogène labile et facultativement un produit chromogène stable,
Im$_2$ correspond à une image avec un produit chromogène labile,
A correspond à une région couverte par l'immunomarquage labile,
O$_i$ correspond à une région couverte par l'immunomarquage stable, et
soit a[m,n] $\in$ A, o[m,n] $\in$ O$_i$ étant des pixels de l'image Im,
et d[m,n], étant le niveau de gris du pixel a[m,n] dans l'image Im$_2$,
alors la densité de surface de l'immunomarquage labile dans l'immunomarquage stable est obtenue par :

$$\frac{|A|}{\sum |O_i|}$$

le niveau de gris moyen d'un sous-ensemble de pixels défini dans l'image *Im$_2$* est défini par :

$$m_o = \frac{\sum_i \sum_{[m,n] \in O_i} d[m,n]}{\sum_i |O_i|}$$

le niveau de gris de pixels dans l'image *Im$_2$* est calculé à partir dudit sous-ensemble de pixels défini dans l'image *Im$_2$* et on y fait référence comme aux pixels dans *Im$_1$,* :

$$m_d = \frac{\sum\limits_{[m,n] \in A} d[m,n]}{|A|} \; .$$

FIG. 1

**Immunohistochemistry** ~35

**Microscopy** ~ 36

**Image processing and analysis** ~ 37

Slide preparation ~ 7

Slide positionning on stage ~ 13

lowpass filtering (11x11 mask) ~28

1st antigen (AbI) detection ~ 8

Landmark definition ~14

Substraction of filtered image from image #1 ~29

1st amplification/ revelation ~ 9

Field positionning — 15

Image #1 acquisition — 16

Threshold segmentation ~30

2nd antigen (AbII) detection ~10

2nd amplification/ revelation ~11

Coverslip ~ 12

17 — Coordinates & image storage

Contour extraction from the resulting binary image ~31

Remove coverslip ~ 19

18 — More fields ?

Masking of image #2 by the binary image ~32

2nd chromogen elution ~20

22 — Slide positionning on stage

SOD measurement in masked image #2 ~33

Coverslip

21

Interactive landmark retrieval — 23

Display SOD value ~34

Field position retrieval — 24

39 — STOP

Online image with contour overlay — 25

More fields ? — 38

Precise positionning — 26

**FIG. 2**

27 — Image #2 acquisition

FIG. 3

I

Ab II: reference

Ab I: density

Ab dilution

## FIG. 4

Acquisition ——— 40

41——— Shading

Density image
Grey level ——— 42

Transformation ———43

Mask image
Binary

44—— Segmentation

45—

## FIG. 5

**FIG. 6A**

**FIG. 6B**

**FIG. 6C**

**FIG. 7A**

**FIG. 7B**

**FIG. 7C**

**FIG. 8**

**FIG. 9**

**FIG. 10**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Xue et al.** *Applied Immunohistochemistry,* 1998, vol. 6 (2), 69-76